# DEMANDE DE BREVET EUROPEEN

(11) **EP 1 291 012 A1**
(43) Date de publication de la demande: **12.03.2003**
(21) Numéro de dépôt: 02292015.1
(22) Date de dépôt: 09.08.2002
(51) Int. Cl.: A61K 7/48, A61K 7/06

(54) **Complexe cosmétique à base d'acide malique**

(30) Priorité: 27.08.2001 FR 0111134
(71) Demandeur: Haarmann & Reimer S.A., 92800 Puteaux (FR)
(72) Inventeur: Bellon, Patrice, 92130 Issy-les-Moulineaux (FR)
(74) Mandataire: Rinuy, Santarelli

(57) **Abrégé**

Un complexe cosmétique comprenant de l'acide malique, de l'extrait d'amande douce et de l'extrait de pulpe de fruit, des compositions cosmétiques renfermant ce complexe ainsi que leurs utilisations dans le domaine de la cosmétique.

## Description

La présente invention concerne un complexe cosmétique comprenant de l'acide malique, de l'extrait d'amande douce et de l'extrait de pulpe de fruit, des compositions cosmétiques renfermant ce complexe ainsi que leurs utilisations dans le domaine de la cosmétique.

Il est connu que les acides organiques ou AHA (alphahydroxyacide), tels que l'acide malique ou l'acide citrique, peuvent être utilisés dans des compositions cosmétiques pour redynamiser la peau et les cheveux et pour apporter du lissage aux cheveux.

Néanmoins, ces AHA possèdent un pH acide conduisant à un potentiel irritant pour la peau et les cheveux lorsqu'ils sont utilisés dans des compositions cosmétiques.

Pour lutter contre l'agression des AHA, il est connu d'utiliser, en association avec ces AHA, des agents adoucissants comme des huiles végétales tels que l'huile d'amande douce et des agents apaisants comme l'allantoïne et l'acide hyaluronique. Toutefois, ces agents s'accumulent sur les cheveux et les alourdissent.

La demanderesse a découvert que l'association d'acide malique, d'extrait d'amande douce et d'extrait de pulpe de fruit permet d'éviter tout ou partie des désavantages et des inconvénients mentionnés ci-dessus.

L'association de ces trois composés présente les avantages surprenants de pouvoir diminuer, arrêter et/ou prévenir l'effet de ternissement des cheveux et du vieillissement de la peau et des cheveux ainsi que d'augmenter l'effet de lissage et de brillance des cheveux, tout en respectant le cuir chevelu et l'intégrité du cheveu.

Le complexe cosmétique de l'invention présente également l'avantage de lisser la peau du visage et du corps en apportant des propriétés hydratantes, sans toutefois provoquer d'irritations.

Les composés du complexe cosmétique de l'invention sont facilement absorbés par les cheveux évitant ainsi leur accumulation à la surface du cheveu et l'alourdissement de la chevelure.

La présente invention a pour premier objet un complexe cosmétique comprenant au moins les trois composés suivants :
a) acide malique,
b) extrait d'amande douce, et
c) extrait de pulpe de fruit.

L'acide malique a) compris dans le complexe cosmétique de l'invention peut provenir de l'extrait de pulpe de fruit c) et/ou de préférence être ajouté aux extraits b) et c). Il est habituellement requis d'avoir une teneur importante en acide malique que l'on ne retrouve généralement pas dans les extraits de pulpes de fruits.

Selon l'invention, l'acide malique total peut représenter de 5 à 40 % en poids, notamment de 5 à 30 % en poids, préférentiellement de 10 à 25 % en poids, encore plus préférentiellement de 15 à 20 % en poids, par rapport au poids total du complexe cosmétique.

Préférentiellement, l'extrait d'amande douce représente de 1 à 30 % en poids, notamment de 1 à 10 % en poids, plus préférentiellement de 2 à 6 % en poids, encore plus préférentiellement de 3 à 5 % en poids, par rapport au poids total du complexe.

Préférentiellement, l'extrait de pulpe de fruit représente de 10 à 60 % en poids, notamment de 10 à 40 % en poids, plus préférentiellement de 15 à 35 % en poids, encore plus préférentiellement de 20 à 30 % en poids, par rapport au poids total du complexe.

Dans le cadre de la présente invention, on entend par "extrait de pulpe de fruit" le produit résultant de l'expression de la pulpe du fruit ou du fruit en entier, tel quel ou sous forme concentré.

Un extrait de pulpe de fruit peut être obtenu selon des méthodes bien connues de l'homme du métier comme par exemple un broyage de la pulpe ou du fruit entier suivi d'une filtration ou une extraction par distillation à l'aide d'un solvant.

De tels extraits sont notamment commercialisés par la société Alban Muller.

L'extrait de pulpe de fruit du complexe cosmétique peut comprendre plusieurs fruits différents. L'extrait de pulpe de fruit peut être obtenu à partir d'un fruit choisi, parmi le pamplemousse, l'orange, le kiwi, la pomme, le melon, le citron, le citron vert, le yuzu, la groseille, la cerise, la poire, la grenade, la pêche, l'abricot, le raisin, le litchi, la fraise, la framboise, l'ananas et la mûre, ou d'un mélange de plusieurs de ces fruits.

Un fruit est toujours entouré d'une péricarpe, appelée aussi l'enveloppe, qui est divisée en trois parties : l'exocarpe, le mésocarpe et l'endocarpe. Dans le cas des fruits appelés drupes, comme la pomme, la poire, la cerise ou la prune par exemple, la pulpe correspond au mésocarpe. Pour les agrumes, le melon, la myrtille ou la courge par exemple, c'est le péricarpe en entier qui correspond à la pulpe. Mais il existe d'autres variantes bien connues de l'homme du métier.

La pulpe de fruit contient généralement des glucides comme des sucres simples (tels que le fructose, le glucose et le saccharose) et des polysaccharides (tels que les pectines, l'amidon et le mucilage), des acides organiques (tel que l'acide malique, l'acide citrique, l'acide lactique et l'acide tartrique), des composés phénoliques comme les flavonoïdes, des terpènes comme les caroténoïdes (tel que le carotène et le lycopène), des vitamines comme les vitamines A et C et des minéraux (tel que le calcium, le potassium, le fer, le sodium, le phosphore et le magnésium).

Préférentiellement, l'extrait de pulpe de fruit peut être obtenu à partir des fruits choisis parmi le pamplemousse (Citrus grandis), l'orange (Citrus aurantium) et le kiwi (Actnidia chinensi).

L'extrait de pulpe de fruit de pamplemousse (Citrus grandis) comporte généralement des glucides tels que des oses et des osides (pectine), des acides organiques tels que l'acide ascorbique (vitamine C), l'acide citrique et l'acide malique, des composés phénoliques, particulièrement des flavonoïdes du type flavanones (hespéridine, naringine), des terpénoïdes tels que des caroténoïdes ainsi qu'une huile essentielle.

Cette huile essentielle est habituellement constituée de :
- composés phénoliques parmi lesquels des coumarines telles que des coumarines simples (esculoside, auraptène, limettine, méranzine) et des furocoumarines (bergaptène), et de
- terpénoïdes tels que des :
   i) monoterpènes : acétate de citronnellyle, citronnellal, géranial, 96 à 98 % de limonène, néral, perillaldéhyde, alpha et beta-sisensal, citral, acétates de géranyle, de béryle, de perillyle, d'octyle, de décyle, de transcarvyle, de 1,8 p-menthadiène-2-yl, et des
   ii) sesquiterpènes : cadinène, paradisiol, intermédéol, des aldéhydes aliphatiques (nonanal, décanal).

L'extrait de pulpe de fruit d'orange (Citrus aurantium) comporte habituellement environ 8 % de glucides, notamment des oses et des osides (pectine, saccharose), des protides tels que des acides aminés (asparagine, glutamine) et des enzymes (peroxydase), des matières minérales tels que du calcium et du potassium, environ 3 % d'acides organiques tels que de l'acide ascorbique (vitamine C), de l'acide citrique, de l'acide malique et de l'acide borique, des composés phénoliques représentés par des phénols tels que des tocophérols et des flavonoïdes de type flavanones : hespéridine et hespérétine, ainsi que des vitamines : vitamine A (rétinol), vitamine B1 (thiamine), vitamine B2 (riboflavine), vitamine B3 (vitamine PP), vitamine B5 (acide pantothénique) et vitamine B6 (pyridoxine).

L'extrait de pulpe de fruit de kiwi (Actnidia chinensi) comporte généralement des acides organiques : acide ascorbique (vitamine C) en forte proportion, des composés phénoliques dont des phénols (tocophérols), de la vitamine B9 (acide folique), une huile qui représente environ 32 % de la graine composée de lipides plus particulièrement des acides gras : environ 16 % d'acide linoléique, environ 63 % d'acide linolénique, environ 13 % d'acide oléique, environ 6 % d'acide palmitique et environ 2 % d'acide stéarique, ainsi que du chrome et du potassium.

La composition de l'extrait d'amande douce (Prunus amygdalus dulcis) est bien connue de l'art antérieur. Cet extrait d'amande douce comporte habituellement environ 6 à 10 % de glucides, environ 20 à 25 % de protides tel que de l'albumine, des acides aminés, un complexe enzymatique (émulsine) contenant de la beta-glucosidase, environ 50 à 60 % de lipides composés de triglycérides constitués d'acides gras dont environ 70 % d'acide oléique (trioléine), environ 15 % d'acide linoléique et d'acide palmitique, environ 0,3 à 1,2 % d'éléments insaponifiables ainsi que des vitamines.

Les éléments insaponifiables renferment des phénols comme le tocophérol (alpha-tocophérol), des triterpènes comme les stéroïdes (betasitostérol) et du squalène.

Les vitamines de l'extrait d'amande douce sont la vitamine A (rétinol), la vitamine B1 (thiamine), la vitamine B2 (riboflavine), la vitamine B3 (vitamine PP, niacine), la vitamine B5 (acide pantothénique) et la vitamine B6 (pyridoxine).

Le complexe selon l'invention comprend généralement, en outre, de l'eau ainsi que des additifs cosmétiquement acceptables comme des conservateurs tels que le phenonip®.

Le phenonip® est un conservateur vendu par la société Nipa, à base de phénoxyéthanol et de mélange de parabens : méthylparaben, butylparaben, éthylparaben, propylparaben et isobutylparaben.

Selon un mode préféré de l'invention, le complexe cosmétique de l'invention contient environ 39,5 % en poids d'eau déionisée, environ 16 % en poids d'acide malique, environ 10 % en poids de butylène glycol, environ 10 % en poids d'extrait de pulpe de pamplemousse, environ 10 % en poids d'extrait de pulpe d'orange, environ 10 % en poids d'extrait de pulpe de kiwi, environ 4 % en poids d'extrait d'amande douce et environ 0,5 % en poids de conservateurs, notamment de phenonip®, par rapport au poids total de la composition.

Le complexe cosmétique est habituellement transparent et d'une couleur jaune. Il ne présente généralement pas d'odeur particulière. Il présente généralement un pH de 6 à 8, de préférence égal ou voisin de 7.

Un complexe selon l'invention peut être utilisé pour la préparation de compositions cosmétiques, qui constituent un autre objet de la présente invention.

La présente invention a ainsi pour autre objet une composition cosmétique comprenant un complexe cosmétique tel que défini précédemment.

Une composition cosmétique selon l'invention comporte habituellement, outre ledit complexe cosmétique, au moins un agent cosmétique tel que de l'eau, un agent tensioactif anionique, non ionique et/ou amphotère, un polymère anionique, non ionique et/ou amphotère, une protéine, un hydrolysat de protéine, une céramide, un pseudocéramide, un acide gras à chaîne linéaire ou ramifiée, un alphahydroxyacide (AHA) autre que l'acide malique, une vitamine, un ester d'acide gras, une silicone volatile ou non volatile, soluble ou non soluble sous forme d'huile, de gomme, de résine et/ou ses homologues organomodifiées, un agents hydratant, un agent antipelliculaire, un agent antiséborrhéique, un agent épaississant, de l'éthanol, un filtre solaire, un agent anti-bactérien, un agent conservateur tel que le butylène glycol, un parfum, un colorant, un agent anti-oxydant, un agent régulateur de pH, un agent anti-transpirant, un agent anti-radicaux libres, une huile minérale, une huile organique et/ou une huile de synthèse.

Les agents tensioactifs anioniques peuvent être de type sulfate comme les sels (en particulier les sels alcalins, notamment de sodium, les sels d'ammonium, les sels d'amines, les sels d'aminoalcools ou les sels de magnésium) des alkylsulfates, alkyléthersulfates, alkylamidoéthersulfates, alkylaryléthersulfate ou alkyléthersulfosuccinates. On utilise de préférence un agent tensioactif anionique choisi parmi les alkyl (C₁₂₋₁₄) éthersulfates de sodium, de triéthanolamine ou d'ammonium oxyéthylénés à environ 2,2 moles d'oxyde d'éthylène.

Les agents tensioactifs anioniques peuvent aussi être de type phosphate, sulfonate et/ou carboxylate comme les alkylsulfonates, alkylphosphates, alkylamidesulfonates, alkylarylsulfonates, α-oléfine-sulfonates, paraffine sulfonates, alkylsulfosuccinates, alkyléthersulfosuccinates, alkylamidesulfosuccinates, alkylsulfosuccinamates, alkylsulfoacétates, alkylétherphosphates, acylsarcosinates, acyliséthionates et les N-acyltaurates.

Les agents tensioactifs non ioniques sont préférentiellement des alkylpolyglycosides, notamment des alkylpolyglucosides.

Les agents tensioactifs amphotères peuvent être, notamment, des dérivés secondaires ou tertiaires aliphatiques, dans lesquels le radical aliphatique est une chaîne linéaire ou ramifiée comportant de 8 à 18 atomes de carbone et contenant au moins un groupe anionique hydrosolubilisant tels qu'un groupe carboxylate, sulfonate, sulfate, phosphate ou phosphonate; ou des alkyl (C₈₋₂₀) bétaïnes, des alkyl (C₈₋₂₀) sulfobétaïnes, des alkyl (C₈₋₂₀) amidoalkyl (C₁₋₆) bétaïnes ou des alkyl (C₈₋₂₀) amidoalkyl (C₁₋₆) sulfobétaïnes.

Les polymères cationiques utilisables peuvent être choisis parmi tous ceux déjà connus en soi comme améliorant les propriétés cosmétiques des cheveux traités par des compositions détergentes.

Une composition cosmétique selon l'invention peut consister en un shampoing, un conditionneur capillaire comme une crème conditionnante pour les cheveux, une crème comme, par exemple, une crème corporelle, une crème hydratante, une crème démêlante pour cheveux, une crème de soin pour les pieds, une crème régénérante et anti-vieillissement, une crème de soin du visage, et une crème pour le soin de la peau des bébés, un lait corporel adoucissant, un baume à lèvre, un gel pour cheveux, un produit anti-solaire, un savon, un gel douche, un sérum pour le visage et le corps et un produit moussant pour la douche et le bain.

Une composition cosmétique selon l'invention peut consister plus particulièrement en un shampooing ou une crème conditionnante pour les cheveux.

Préférentiellement, le complexe cosmétique de l'invention représente entre 0,1 et 20 % en poids, préférentiellement entre 0,5 et 15 % en poids, du poids total de la composition cosmétique.

La présente invention a également pour objet un procédé de préparation d'un complexe cosmétique selon l'invention, comprenant les étapes suivantes:
a) on mélange au moins de l'extrait d'amande douce et de l'extrait de pulpe de fruit et optionnellement de l'acide malique; et
b) on récupère le complexe cosmétique obtenu.

Préférentiellement, on rajoute de l'acide malique à l'étape a).

La présente invention a aussi pour objet un procédé de préparation d'une composition cosmétique selon l'invention, comprenant les étapes suivantes:
a) on prépare un complexe cosmétique en mélangeant au moins de l'extrait d'amande douce et de l'extrait de pulpe de fruit et optionnellement de l'acide malique;
b) on ajoute au moins un agent cosmétique tel que défini précédemment dans le complexe cosmétique a); et
c) on récupère la composition cosmétique obtenue.

Préférentiellement, on rajoute de l'acide malique à l'étape a).

Le complexe cosmétique de l'invention, en tant que tel, peut lui-même servir de composition cosmétique.

L'extrait d'amande douce et l'extrait de pulpe de fruit et optionnellement de l'acide malique peuvent être mélangés à température ambiante pendant 15 minutes sous une agitation moyenne dans un mélangeur planétaire.

La présente invention a également pour objet un procédé cosmétique de traitement de la peau, des poils et/ou des cheveux, caractérisé en ce qu'il consiste à appliquer sur la peau, les poils et/ou les cheveux une composition cosmétique telle que définie précédemment.

La présente invention a encore pour objet l'utilisation d'un complexe cosmétique ou d'une composition cosmétique selon l'invention pour le traitement de la peau, des poils et/ou des cheveux.

Le complexe ou la composition cosmétique peut notamment être utilisé pour diminuer, arrêter et/ou prévenir l'effet terne des cheveux, pour diminuer, arrêter et/ou prévenir le vieillissement de la peau et des cheveux et/ou pour augmenter l'effet de lissage et de brillance des cheveux.

### EXEMPLES

### Exemple 1 : Complexe cosmétique

| **COMPOSES** | **% EN POIDS** | **FOURNISSEURS** |
|---|---|---|
| Eau déionisée | 39,5 | |
| Acide malique | 16 | Merck |
| Butylène glycol | 10 | Brenntag |
| Extrait de pulpe de pamplemousse | 10 | Alban Muller |
| Extrait de pulpe d'orange | 10 | Alban Muller |
| Extrait de pulpe de kiwi | 10 | Alban Muller |
| Extrait d'amande douce | 4 | Alban Muller |
| Phenonip ® (conservateur à base de phénoxyéthanol et de mélange de parabens) | 0,5 | Nipa |
| Le complexe cosmétique à un pH de 7 +/- 0,20. | | |

### Exemple 2 : Shampooing

| Phase 1 | | |
|---|---|---|
| **COMPOSES** | **% EN POIDS** | **FOURNISSEURS** |
| Eau déionisée | 36,16 | |
| Texapon NSO IS ® (sulfate laureth de sodium) | 15 | Cognis |
| Tegobetaïne ® (bétaîne de cocamidopropyle) | 30 | Goldschmidt |
| Sarcosinate CT-30 ® (cocoylsarcosinate de triéthylamonnium) | 8,5 | Jan Dekker |
| EDTA 3 Na 2H₂O (EDTA trisodique) | 0,1 | TriK |
| Phenonip® (conservateur à base de phénoxyéthanol et de mélange de parabens) | 1 | Nipa |
| Aculyn 22 ® (acrylates steareth-20 copolymère de méthacrylate) | 7 | ISP |
| Complexe de l'exemple 1 | 1 | |
| Hydroxyde de potassium | 0,24 | Merck |

| Phase 2 | | |
|---|---|---|
| **COMPOSES** | **% EN POIDS** | **FOURNISSEURS** |
| Solubilisant LRI ® (PPG 26 buteth 26 & PEG 40 hydrogéné, huile de castor) | 0,5 | Wackherr |
| Parfum (fragrance) | 0,5 | |

Les composés de la phase 1 sont mélangés à température ambiante. Les composés de la phase 2 sont mélangés à 35°C.

La phase 1 et la phase 2 sont mélangées jusqu'à obtenir une solution homogène. Le shampooing obtenu est transparent.

### Exemple 3 : Crème conditionnante

| Phase 1 | | |
|---|---|---|
| **COMPOSES** | **% EN POIDS** | **FOURNISSEURS** |
| Eau distillée | 83,8 | |
| Lexquat AMG-BEO ® (chlorure de behenamidopropyl-PG dimomium) | 0,3 | Inolex |

| Phase 2 | | |
|---|---|---|
| **COMPOSES** | **% EN POIDS** | **FOURNISSEURS** |
| Hyfatol CS ® (alcool cétéarylique) | 5 | Aarhus |
| Lexamine S13 ® (diméthylamine stéaramidopropyle) | 0,4 | Inolex |
| Paramul J ® (alcool cétéarylique et cétéareth 20) | 3 | Bernel |

| Phase 3 | | |
|---|---|---|
| **COMPOSES** | **% EN POIDS** | **FOURNISSEURS** |
| Mirasil C-DPDM ® (cyclométhicone et diphénylciméthycone) | 1,5 | Rhodia |

| Phase 4 | | |
|---|---|---|
| **COMPOSES** | **% EN POIDS** | **FOURNISSEURS** |
| Lexquat AMG-BEO ® (chlorure de behenamidopropyl-PG dimomium) | 3 | Inolex |

| Phase 5 | | |
|---|---|---|
| **COMPOSES** | **% EN POIDS** | **FOURNISSEURS** |
| Phenonip ® (conservateur à base de phénoxyéthanol et de mélange de parabens) | 1 | Nipa |
| Complexe de l'exemple 1 | 1 | |

| Phase 6 | | |
|---|---|---|
| **COMPOSES** | **% EN POIDS** | **FOURNISSEURS** |
| Parfum (fragrance) | 1 | Merck Eurolab |
| Acide citrique 50 % | Jusqu'à pH=5,2 | |

La phase 1 est obtenue en mélangeant les composés à une température de 80°C environ jusqu'à homogénéisation. La phase 2 est obtenue en mélangeant les composés à une température de 80°C environ jusqu'à homogénéisation.

On mélange les phases 1 et 2 à une température de 50°C.

On ajoute ensuite la phase 3, sous agitation, à une température de 45°C. On ajoute alors la phase 4, sous agitation, à une température de 45°C.

Enfin, à une température de 35°C on ajoute les phases 5 et 6, sous agitation, jusqu'à homogénéisation.

### Exemple 4 : Test comparatif

Dans le test comparatif ci-dessous on a comparé, sur les cheveux, l'effet d'un shampooing et d'une crème conditionnante conforme ou non à l'invention.

Le shampooing et la crème de référence correspondent respectivement aux produits des exemples 2 et 3 ne comprenant pas le complexe cosmétique de l'exemple 1.

Le shampooing et la crème conforme à l'invention correspondent respectivement aux produits des exemples 2 et 3.

Sur un premier panel de 10 personnes on applique pendant 1 minute le shampooing conforme à l'invention et on rince.

On applique ensuite la crème conditionnante conforme à l'invention et on rince.

On sèche ensuite les cheveux.

Sur un deuxième panel de 10 personnes on applique pendant 1 minute le shampooing de référence et on rince.

On applique ensuite la crème conditionnante de référence et on rince.

On sèche ensuite les cheveux.

On observe alors au microscope l'aspect du cheveu.

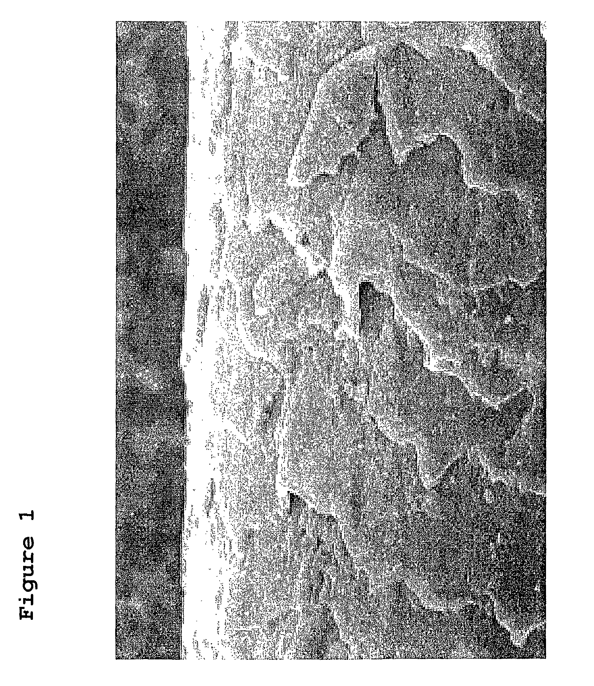

La Figure 1 montre l'aspect d'un cheveu traité avec le shampooing et la crème de référence. On observe que le cheveu est rugueux et endommagé.

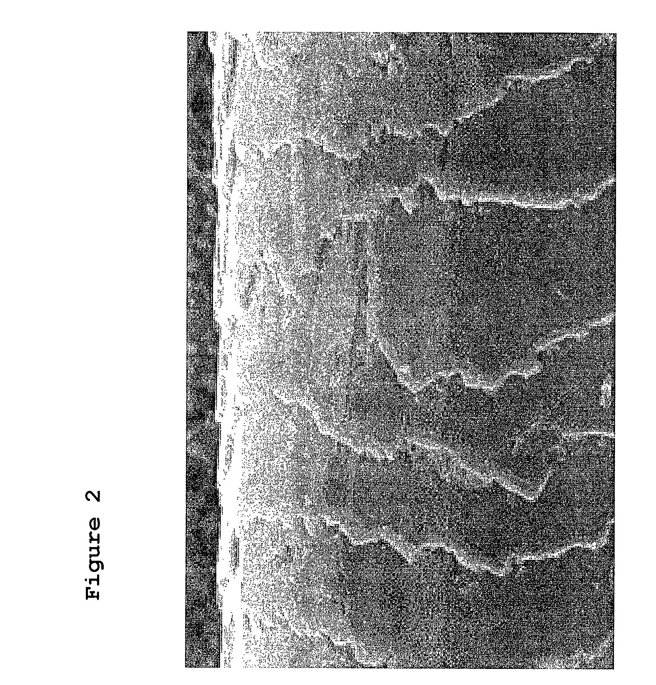

La Figure 2 montre l'aspect d'un cheveu traité avec le shampooing et la crème conformes à l'invention (exemples 2 et 4). On observe que le cheveu est lisse et que la fibre capillaire est gainée.

Ainsi, le shampooing et la crème comprenant la composition cosmétique de l'invention permet de donner aux cheveux des propriétés de lissage et d'anti-ternissement tout en respectant le cuir chevelu.

## Revendications

1. Complexe cosmétique comprenant les composés suivant :
a) acide malique,
b) extrait d'amande douce, et
c) extrait de pulpe de fruit.

2. Complexe selon la revendication 1, **caractérisé en ce que** l'acide malique représente de 5 à 40 % en poids, par rapport au poids total du complexe.

3. Complexe selon l'une quelconque des revendications 1 à 2, **caractérisé en ce que** l'extrait d'amande douce représente de 1 à 30 % en poids, par rapport au poids total du complexe.

4. Complexe selon l'une quelconque des revendications 1 à 3, **caractérisé en ce que** l'extrait de pulpe de fruit représente de 10 à 60 % en poids, par rapport au poids total du complexe.

5. Complexe selon l'une quelconque des revendications 1 à 4, **caractérisé en ce que** l'extrait de pulpe de fruit est obtenu à partir d'au moins un fruit choisi parmi le pamplemousse, l'orange, le kiwi, la pomme, le melon, le citron, le citron vert, le yuzu, la groseille, la cerise, la poire, la grenade, la pêche, l'abricot, le raisin, le litchi, la fraise, la framboise, l'ananas et la mûre.

6. Complexe selon l'une quelconque des revendications 1 à 5, **caractérisé en ce qu'**il contient environ 39 % en poids d'eau déionisée, environ 16 % en poids d'acide malique, environ 10 % en poids de butylène glycol, environ 10 % en poids d'extrait de pulpe de pamplemousse, environ 10 % en poids d'extrait de pulpe d'orange, environ 10 % en poids d'extrait de pulpe de kiwi, environ 4 % en poids d'extrait d'amande douce et environ 1 % en poids de phenonip®, par rapport au poids total du complexe.

7. Composition cosmétique **caractérisée en ce qu'**elle comprend un complexe cosmétique selon l'une quelconque des revendications 1 à 6.

8. Composition cosmétique selon la revendication 7, **caractérisée en ce qu'**elle comprend au moins un agent cosmétique tel que de l'eau, un agent tensioactif anionique, non ionique et/ou amphotère, un polymère anionique, non ionique et/ou amphotère, une protéine, un hydrolysat de protéine, une céramide, un pseudocéramide, un acides gras à chaînes linéaires ou ramifiées, un alphahydroxyacide (AHA), une vitamine, un ester d'acide gras, une silicone volatile ou non volatile, soluble ou non soluble sous forme d'huile, de gomme, de résine et/ou ses homologues organomodifiées, un agents hydratant, un agent antipelliculaire, un agent antiséborrhéique, un agent épaississant, de l'éthanol, un filtre solaire, un agent anti-bactérien, un agent conservateur tel que le butylène glycol, un parfum, un colorant, un agent anti-oxydant, un agent régulateur de pH, un agent anti-transpirant, un agent anti-radicaux libres, une huile minérale, une huile organique et/ou une huile de synthèse.

9. Composition cosmétique selon l'une quelconque des revendications 7 à 8, **caractérisée en ce qu'**elle consiste en un conditionneur capillaire, une crème comme, par exemple, une crème corporelle, une crème hydratante, une crème démêlante pour cheveux, une crème de soin pour les pieds, une crème régénérante et anti-vieillissement, une crème de soin du visage, et une crème pour le soin de la peau des bébés, un lait corporel adoucissant, un baume à lèvre, un gel pour cheveux, un produit anti-solaire, un savon, un gel douche, un sérum pour le visage et le corps et un produit moussant pour la douche et le bain.

10. Composition cosmétique selon l'une quelconque des revendications 7 à 8, **caractérisée en ce qu'**elle consiste en un shampoing et une crème conditionnante pour les cheveux.

11. Composition cosmétique selon l'une quelconque des revendications 7 à 10, **caractérisée en ce que** le complexe cosmétique selon l'une quelconque des revendications 1 à 6 représente entre 0,1 et 20 % en poids du poids total de la composition cosmétique.

12. Procédé de préparation d'un complexe cosmétique selon l'une quelconque des revendications 1 à 6, comprenant les étapes suivantes:
a) on mélange au moins de l'extrait d'amande douce et de l'extrait de pulpe de fruit et optionnellement de l'acide malique; et
b) on récupère le complexe cosmétique obtenu.

13. Procédé de préparation d'un complexe cosmétique selon la revendication 12, **caractérisé en ce que** l'on ajoute de l'acide malique à l'étape a).

14. Procédé de préparation d'une composition cosmétique selon l'une quelconque des revendications 7 à 11, comprenant les étapes suivantes :
a) on prépare un complexe cosmétique en mélangeant au moins de l'extrait d'amande douce et de l'extrait de pulpe de fruit et optionnellement de l'acide malique;
b) on ajoute au moins un agent cosmétique tel que défini dans la revendication 8; et
c) on récupère la composition cosmétique obtenue.

15. Procédé de préparation d'une composition cosmétique selon la revendication 14, **caractérisé en ce que** l'on ajoute de l'acide malique à l'étape a).

16. Procédé cosmétique de traitement de la peau, des poils et/ou des cheveux, **caractérisé en ce qu'**il consiste à appliquer sur la peau, les poils et/ou les cheveux une composition cosmétique selon l'une quelconque des revendications 7 à 11.

17. Utilisation de la composition cosmétique selon l'une quelconque des revendications 7 à 11, pour le traitement de la peau, des poils et/ou des cheveux.

18. Utilisation selon la revendication 17, pour diminuer, arrêter et/ou prévenir l'effet terne des cheveux.

19. Utilisation selon la revendication 17, pour diminuer, arrêter et/ou prévenir le vieillissement de la peau et des cheveux.

20. Utilisation selon la revendication 17, pour augmenter l'effet de lissage et de brillance des cheveux.
